# EUROPEAN PATENT SPECIFICATION

(11) **EP 3 772 646 B1**
(45) Date of publication and mention of the grant of the patent: **24.04.2024**
(21) Application number: 19190268.3
(22) Date of filing: 06.08.2019
(51) Int. Cl.: G01N 21/84

(54) **TEST STRIP, MONITORING DEVICE AND METHOD FOR FABRICATING A TEST STRIP**
TESTSTREIFEN, ÜBERWACHUNGSVORRICHTUNG UND VERFAHREN ZUR HERSTELLUNG EINES TESTSTREIFENS
BANDE DE TEST, DISPOSITIF DE SURVEILLANCE ET PROCÉDÉ DE FABRICATION D'UNE BANDE DE TEST

(43) Date of publication of application: 10.02.2021
(73) Proprietor: ams-OSRAM AG, 8141 Premstätten (AT)
(72) Inventor: Manzi, Giuliano, 8141 PREMSTÄTTEN (AT)
(74) Representative: Epping - Hermann - Fischer

(56) References cited:
- EP-A1- 1 582 598
- WO-A1-2008/098722
- US-A1- 2005 227 371
- US-A1- 2006 132 786
- US-A1- 2015 241 455
- CAPUTO D ET AL: "Smart thin layer chromatography plate", LAB ON A CHIP, ROYAL SOCIETY OF CHEMISTRY, vol. 7, 9 July 2007 (2007-07-09), pages 978-980, XP002484937, ISSN: 1473-0197, DOI: 10.1039/B709145A

## Description

The present disclosure is related to a test strip, a monitoring device and a method for fabricating a test strip.

A test strip is a part of a monitoring device. The part can be inserted before a test or measurement and removed after the test or measurement. The monitoring device is typically portable and, thus, can be used for point-of-care applications for medical diagnostics and environmental tests. The test strip can be used for a lateral flow test. The test strip uses the capillary action of a porous material and the ability of the porous material to bind marker molecules.

A sample liquid such as water, urine, blood or another liquid is provided to the test strip. The sample liquid flows using the capillary effect of the porous material and performs a chemical reaction. Typically, the chemical reaction results in a change of color at a predetermined active area of the porous material. The active area may have the form of a narrow or broad line. Often there are reactions at two active areas, respectively lines, of the porous material. Typically, change of color is monitored by visual inspection. These class of tests have several advantages, but there are still some disadvantages related e.g. to sensitivity and multi-analyte detection. Finding a solution for these drawbacks may bring more tests to a home environment.

WO 2008/098722 A1 discloses a transillumination measurement device for determining the intensity of a chemical/biological reaction, comprising at least one carrier layer, a light source, a quantum detector of electromagnetic radiation, and an interface. A reaction membrane is applied to the carrier layer and features at least one chemically/biologically reactive test area. The electromagnetic radiation emitted from the light source lies in a first optical spectral range that overlaps a second optical spectral range of the quantum detector. The reaction membrane is arranged on a first flat face of the carrier layer, while the quantum detector is arranged on a second flat face of the carrier layer.

US 2015/241455 A1 discloses an apparatus for identifying at least a first target condition in a human or animal body. The apparatus comprises one or more test portions for identifying a first analyte in a biological sample from the body. According to an embodiment, an LED is configured to emit light that is incident on a test stripe. A photodetector is capable of monitoring light reflection or light output at one or more test portions located on the test stripe. Multiple analytes may be detected by a multi-wavelength photodetector.

EP 1582598 A1 discloses a rapid diagnostic test system using a single-use module that includes a photodetector. The photodetector generates an electrical signal representing a measurement of light from a test region on a medium such as a lateral-flow strip for a binding assay. The test medium contains a labeling substance that attaches a persistent fluorescent structure to a target analyte, so that the photodetector measures fluorescent light. Multiple photodetectors allow simultaneous testing for multiple analytes with different fluorescent labeling substances. The electrical signal can be output for processing in a reusable module.

It is an object to provide a test strip, a monitoring device and a method for fabricating a test strip that allow an efficient read-out of measurement results.

These objects are achieved by the subject-matter of the independent claims. Further developments and embodiments are described in the dependent claims.

According to the invention, a test strip comprises a porous material, a photodetector and a substrate with a first and a second side. The porous material is attached to the first side of the substrate. The photodetector is attached to the second side of the substrate. The photodetector is implemented as a spectral sensor that is configured to separately detect light in at least two different wavelength regions. The test strip is free from a light source.

Advantageously, the porous material and the photodetector are both fixed at the substrate during production of the test strip. Advantageously, a distance from the photodetector to the porous material is short and the location of the photodetector is predetermined with respect to the porous material resulting in a high efficiency and reproducibility for detecting light transmitted or emitted by the porous material.

In an embodiment, the test strip comprises conducting lines arranged at the second side of the substrate. The photodetector comprises contact areas which are electrically connected to the conducting lines at the second side of the substrate. Advantageously, the substrate with the conducting lines can provide electrical power and signals to the photodetector. The only electrical connections to the photodetector run over the conducting lines on the substrate.

In an embodiment, the test strip comprises an anisotropic conducting film that mechanically connects the photodetector to the second side of the substrate and electrically connects the contact areas of the photodetector to the conducting lines at the second side of the substrate. Advantageously, the anisotropic conducting film allows a stable fixation of the photodetector on the substrate with low thickness. Advantageously, the anisotropic conducting film realizes a conductive connection of one contact area to one of the conducting lines and isolates this contact area to the other conducting lines. Advantageously, the electrical connection of the photodetector is free of a bond wire. The anisotropic conducting film acts as an adhesive underfiller.

In an embodiment, the conducting lines comprise a power supply line, a reference potential line and at least one bus line. Advantageously, electrical power can be provided to the photodetector from a power supply using the power supply line and the reference potential line. Data can be transmitted from the photodetector to other circuits of a monitoring device via the at least one bus line.

The porous material may comprise nitrocellulose. The porous material may be bibulous. The porous material may be covered by a film. The film may protect the porous material. The film may be transparent or translucent. Transparent may be named also optically clear. The porous material may have a specific ratio of pore size, porosity and thickness plus a dedicated chemical treatment.

In an embodiment, the substrate is transparent or translucent. Thus, light emitted or transmitted from the porous layer can reach the photodetector via the substrate.

In an alternative embodiment, the substrate is opaque and comprises a transparent or a translucent area. The transparent or translucent area may be realized as a window of the substrate. The photodetector is located at this transparent or translucent area.

The porous material, in particular comprising nitrocellulose, is operable to transfer a liquid from an input point of the porous material to an active area of the porous material. The active area of the porous material is provided with a chemical substance, typically a chemical compound, which reacts with a component of the liquid. The component may be an analyte included in the liquid. The photodetector is arranged adjacent to the active area.

In an embodiment, the porous material comprises at least two active areas. Thus, two components of the liquid or two analytes may be detected at the at least two active areas.

In an embodiment, the photodetector comprises at least two pixels which are arranged adjacent to the at least two active areas. A pixel can be named photodetector element. A pixel may be realized as photodiode.

In an alternative embodiment, the photodetector is arranged adjacent to one of the at least two active areas. An additional photodetector of the test strip is arranged adjacent to another one of the at least two active areas.

According to the invention, the photodetector is implemented as a spectral sensor that is operable to detect light in at least two different wavelength regions, in particular to separately detect light in at least two different wavelength regions. Two colors are generated at one active area; each of the colors are measured separately by the spectral sensor. Thus, two components or two analytes are detected at one active area. The spectral sensor is operable to detect light in more than two, more than four or more than eight different wavelength regions. The different wavelength regions may be in the visible range or in the visible plus infrared range or in the visible plus near-infrared range.

In an embodiment, the active area may have the form of a line, a square, a rectangle, a dot, a circle or an ellipse. The line may be narrow or broad.

In an embodiment, the active areas may each have the form of a line, a square, a rectangle, a dot, a circle or an ellipse. The active areas may be arranged like a matrix, e.g. as a matrix of dots or squares. The active areas may have different colors or may have identical colors. At least two active areas may have different colors. At least two active areas may have identical colors.

In an embodiment, the test strip comprises a sample pad for absorbing liquid and providing the liquid to the porous material directly or via a conjugate pad of the test strip. The conjugate pad includes e.g. a chemical substance (typically a chemical compound) designed for reaction with an analyte included in the liquid.

In an embodiment, the test strip comprises an absorbing pad for absorbing excess liquid from the porous material.

In an embodiment, the sample pad, the conjugate pad and the absorbing pad are also realized by a porous film or a porous layer, such as made of nitrocellulose.

In an embodiment, a monitoring device comprises the test strip, a light source and a control circuit connected to the photodetector and to the light source. The porous material and the substrate are located between the light source and the photodetector. The monitoring device may be named reader.

In an embodiment, the monitoring device is operable such that the test strip is inserted into the device and removed again. Thus, the test strip is used once, namely for a single test.

The monitoring device is designed to be used several times, e.g. with different test strips for detecting different analytes or with test strips for detecting the same analyte.

In an embodiment, the control circuit is operable to detect whether the test strip is inserted or not and to provide an enable signal when the test strip is inserted. Advantageously, by generating the enable signal, the monitoring device may be able to detect the correct insertion of the test strip into the monitoring device or/and may be able to detect whether the correct test strip is inserted.

In an embodiment, the monitoring device is operable to perform a lateral flow test, abbreviated LFT, or a lateral flow immunochromatographic assay.

The invention is also directed to a method for fabricating a test strip according to claim 12.

Advantageously, the attachment of the porous material and of the photodetector on the two sides of the substrate results in a compact and stable stack which allows a high accuracy of measurement of light provided or transmitted by the porous material.

In an embodiment, the photodetector is attached to the second side of the substrate by flip-chip assembly. Thus, a reliable connection of the photodetector to the substrate is achieved. In an embodiment, an anisotropic conducting film is provided between the photodetector and the second side of the substrate. Advantageously, a lead frame or a bond wire is not required for electrically contacting the photodetector.

The method for fabricating a test strip may be implemented, for example, by the test strip and the monitoring device according to one of the embodiments defined above.

The test strip with embedded spectral sensor is configured for an optical assay reading device. The test strip is implemented as a smart test strip. The smart test strip is with an embedded spectral sensor. The test strip is configured for a lateral flow test system, abbreviated as LFT system. The LFT system performs refracted and/or absorbance measurements.

The disclosure applies to the field of lateral-flow-test for point-of-care (abbreviated PoC). The test strip reacts to a certain substance that is present in the liquid under test, and color of the porous active area, realized e.g. by depositing conjugated antibodies on the nitrocellulose membrane, changes accordingly when analyte bind to conjugated antibodies. The liquid under test may be named sample, sample liquid or analyte. Alternatively, the substance to be detected is called analyte. The substance to be detected may be a chemical element or a chemical compound.

An example of an application is a home pregnancy test. The test is e.g. able to detect human chorionic gonadotropin (HCG) in urine of a pregnant women. The test assay utilizes the capillary action of porous paper and the ability to bind marker proteins to the cellulose. Usually a two line pattern is used. The first line generates a yes/no signal (pregnant or not). The second line indicates if the test is successful or not. Point-of-care tests (PoC tests) have the ability to test a patient at the point where the care is necessary. This allows a faster diagnosis, hence a faster treatment.

Normally the LFT is read (analyzed) by the human eye, and therefore the ability is lacking to measure variation in concentrations accurately. Lateral flow tests also known as lateral flow immunochromatographic assays are effective devices intended to detect the presence (or absence) of a target analyte in a sample (matrix) without the need for specialized and costly equipment, though many lab based applications exist that are supported by reading equipment. Typically, these tests are used for medical diagnostics either for home testing, point of care testing or laboratory use.

Advantageously, the test strip described in the present disclosure aims at an improvement of system performance, e.g. a further increase of the reading accuracy and better quantitative analysis by an improved location of the spectral sensor with respect to the membrane area that needs to be analysed. Moreover, the LFT PoC reading device may be improved, e.g. by a cost reduction on the reader side.

The following description of figures of embodiments may further illustrate and explain aspects of the test strip, the monitoring device and the method for fabricating a test strip. Devices, areas and layers with the same structure and the same effect, respectively, appear with equivalent reference symbols. Insofar as devices, areas and layers correspond to one another in terms of their function in different figures, the description thereof is not repeated for each of the following figures.
Figure 1 shows an example of a test strip;
Figures 2A to 2D show examples of details of a test strip;
Figures 3A and 3B show further examples of a test strip
Figures 4A to 4C show additional examples of a test strip and of a monitoring device; and
Figures 5A to 5D show simulation results, wherein the example of Figure 5A does not form part of the present invention.

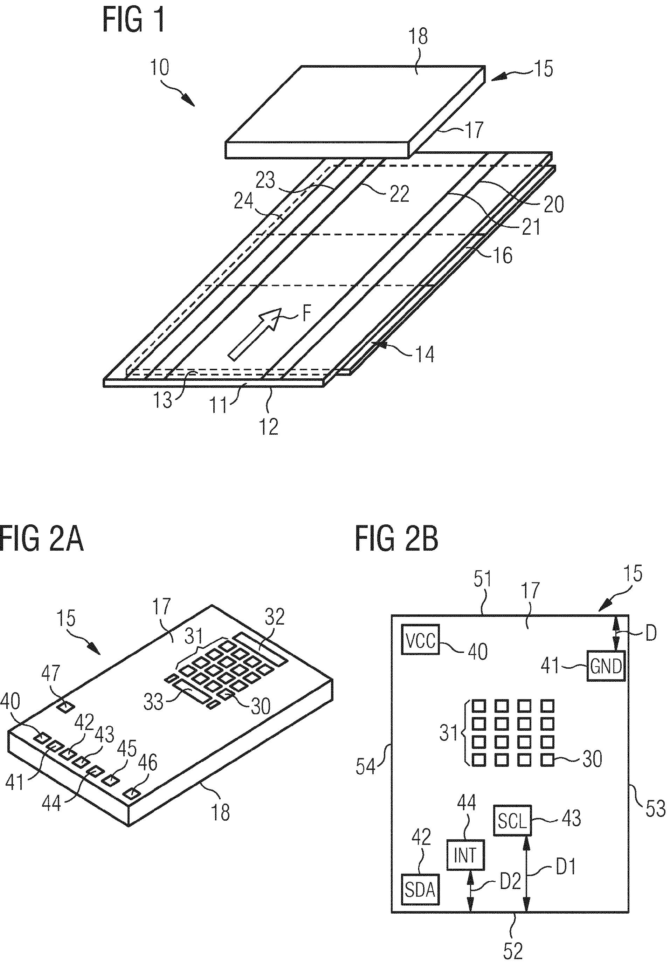

Figure 1 shows an example of a test strip 10. The test strip 10 comprises a substrate 11 with a first and a second side 12, 13, a porous material 14 and a photodetector 15. In Figure 1, a sketch of a possible assembly layout of the test strip 10 is elucidated. The porous material 14 is attached to the first side 12 of the substrate 11. The photodetector 15 is attached to the second side 13 of the substrate 11. The porous material 14 may be directly and permanently connected to the first side 12 of the substrate 11. The substrate 11 is translucent or transparent. The substrate 11 may be realized as a printed circuit board, abbreviated PCB. The substrate 11 is made of plastic or a polymer such as polystyrene, polyethylene, polyethylene terephthalate (abbreviated PET), vinyl, polyester, polyimide, acrylamide, epoxy resin or a woven fiberglass cloth impregnated with an epoxy resin (usually named FR-4 glass epoxy) or glass.

The substrate 11 may have a thickness between 20 µm to 200 µm or between 50 µm to 100 µm or between 60 µm to 90 µm. For example, a PET based substrate 11 may have a thickness out of these ranges. A PET based substrate 11 is transparent and is well suitable for copper deposition and/or etching for conductive traces placement. A PET based substrate 11 works well for direct flip chip attach.

Alternatively, the substrate 11 includes a PCB based material. Such a PCB based substrate 11 is suitable. Optionally, the substrate 11 includes at least a window or via corresponding to the area of the photodetector 15 or to the area of the pixels or pixels of the photodetector 15 (because normally a PCB is are not very transparent).

Correspondingly, the photodetector 15 may be directly and permanently connected to the second side 13 of the substrate 11.

The porous material 14 is translucent or transparent. The porous material 14 has the form of a layer, membrane, film or sheet. The porous material 14 may be made of nitrocellulose. The porous material 14 may be fabricated as a nitrocellulose membrane. The porous material 14 may have an active area 16. The porous material 14 is configured such that a liquid can laterally flow in the porous material 14. The direction of flow F is indicated by an arrow. The flow F of the liquid is performed using a capillary effect in the porous material 14. The liquid may be named sample liquid.

The photodetector 15 is implemented as a spectral sensor. The photodetector 15 may be fabricated as a spectral sensor integrated circuit. Since the pixels and contact areas of the photodetector 15 are on a first side 17 of the photodetector 15 which is a bottom side of the photodetector 15, the pixels and contact areas cannot be seen in the view shown in Figure 1. The photodetector 15 has a second side 18 shown in Figure 1.

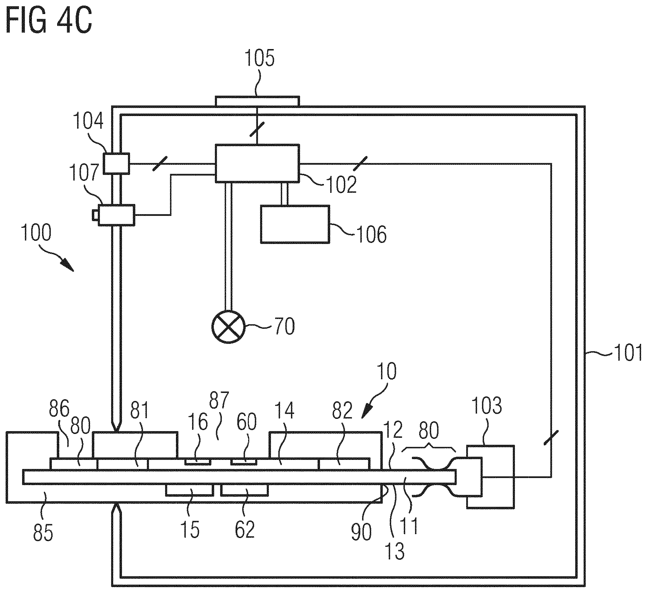

The test strip 10 comprises conducting lines 20 to 24 arranged at the second side 13 of the substrate 11. Thus, the conducting lines 20 to 24 face the first side 17 of the photodetector 15. The conducting lines 20 to 24 can also be named "traces". The conducting lines 20 to 24 are made out of a metal such as copper, aluminum or silver (e.g. fabricated as printed silver ink). The conducting lines 20 to 24 made out of copper or aluminum may be etched. The conducting lines 20 to 24 may be designed partially as straight lines and partially as curved lines. The conducting lines 20 to 24 may run parallel. The conducting lines 20 to 24 are aligned with contact areas of the photodetector 15 (e.g. as shown in Figures 2A and 2B) and conductive pads at the end of the test strip 10 (to mechanically align with a strip holder or socket e.g. as shown in Figure 4C). The conducting lines 20 to 24 may run parallel to the arrow indicating the direction of the flow F of the liquid. The conducting lines 20 to 24 may be e.g. rectangular. For example, a first conducting line 20 may be realized as a power supply line. A second conducting line 21 may be implemented as a reference potential line. A third line 22 may be designed as a bus line. A fourth and/or a fifth conducting line 23, 24 may also be realized as bus lines. Thus, the conducting lines 20 to 24 comprise at least one bus line 22 to 24. The bus lines 22, 23 may be realized as inter-integrated circuit bus lines, abbreviated I²C lines.

Figure 2A shows an example of the photodetector 15 that can be inserted in the test strip 10 shown in Figure 1. In Figure 2A, a perspective view on the photodetector 15 is illustrated. The photodetector 15 comprises at least one pixel 30 on the first side 17 of the photodetector 15. The pixel 30 may be called photodetector element. The photodetector 15 may comprise a photodetector array 31 of pixels 30 on the first side 17 of the photodetector 18. The photodetector array 31 may be an n · m array of pixels. In the example shown in Figure 2A, the photodetector array 31 is a 4 . 4 array. The pixels 30 of the photodetector array 31 may be sensitive for different regions of light. The pixels 30 may be realized as photodiodes. Thus, the photodetector array 31 realizes a spectral sensor. Additionally, the photodetector 15 may comprise an additional pixel 32 and a further pixel 33 that are larger than a pixel 30 of the photodetector array 31 and are located adjacent to the photodetector array 31.

Moreover, the photodetector 15 comprises contact areas 40 to 47 on the first side 17 of the photodetector 15. The contact areas 40 to 47 are arranged at a border or two borders of the photodetector 15. The contact areas 40 to 46 have a distance to the photodetector array 31. The contact areas 40 to 46 can be realized as bond pads or chip bumps.

Figure 2B shows another example of the photodetector 15 that is a further development of the photodetector 15 shown in Figures 1 and 2A. The photodetector array 31 is in the center or nearly in the center of the photodetector 15. A first contact area 40 is realized as a power supply contact area for receiving a supply voltage VCC. A second contact area 41 is implemented as reference potential contact area for receiving a reference potential GND. At least one contact area 42 is designed as a bus contact area. For example, a third and a fourth contact area 42, 43 are implemented as bus contact areas, for example for an inter-integrated circuit bus, abbreviated I²C bus. For example the third and the fourth contact area 42, 43 receive signals or provides signals of the I²C bus such as a data signal SDA and a clock signal SCL. The fifth contact area 44 may be designed for a bus or for another purpose and receives a signal INT.

The contact areas 40 to 44 are not arranged at exactly one border of the photodetector 15. The photodetector 15 has a first to a fourth border 51 to 54. The first border 51 is opposite to the second border 52. The first border 51 runs parallel to the second border 52. The third border 53 is opposite to the fourth border 44. The third border 53 runs parallel to the fourth border 54. The first contact area 40 may be located at the first border 51. The second contact area 41 may be located near the first border 51 but has a distance D to the first border 51. The first contact area 40 has a distance smaller than the distance D to the first border 51.

The third contact area 42 is located adjacent to the second border 52. The fourth and the fifth contact area 43, 44 are adjacent to the second border 52 and have distances D1, D2 to the second border 52. The third contact area 42 has a distance smaller than the distances D1 and D2 to the second border 52. The fourth contact area 43 may have the distance D1 to the second border 52 and the fifth contact area 44 may have the distance D2 to the second border 52. The distance D1 may be larger than the distance D2. Advantageously, the contact areas 40 to 44 are distributed between the first border 51 and the second border 52. The photodetector 15 is free of a contact area that has the same or approximately the same distance to the first border 51 as any other contact area. The contact areas 40 to 44 have different distances to the first border 51 and, thus, also to the second border 52. At least two of the contact areas 40 to 44 have different distances also to the third border 53 and, thus, also to the fourth border 54.

Thus, when looking also at Figure 1, the first side 17 of the photodetector 15 can be attached to the second side 13 of the substrate 11 such that the first contact area 40 is in contact with the first conducting line 20 and has a distance to each other conducting line 21 to 24. Thus, there is only an electrical contact of the first contact area 40 to the first conducting line 20. The first contact area 40 is isolated from the other conducting lines 21 to 24. Correspondingly, the second contact area 42 is only in contact with the second conducting line 21. Similarly, the third to the fifth contact areas 42 to 44 are in electrical contact with their respective conducting lines 22 to 24. Consequently, each of the contact areas 40 to 44 is in mechanical and electrical contact only to exactly one conducting line and is isolated from the other conducting lines. Thus, the conducting lines 20 to 24 may be realized as metal lines. The conducting lines 20 to 24 may be fabricated without an isolation layer on top of the conducting lines 20 to 24 which comprises a contact opening for an electrical contact to the respective contact areas.

Advantageously, the locations of the contact areas 40 to 44 on the first side 17 of the photodetector 15 allow an easy contacting of the conducting lines 20 to 24 on the second side 13 of the substrate 11.

The photodetector 15 is realized as a spectral sensor. The photodetector 15 may have a layout optimized for direct bonding (or any other bonding process). The photodetector 15 may have a layout optimized for direct flip chip attach.

In an alternative embodiment, the conducting lines 20 to 24 are covered by an isolating layer that has openings at the respective locations for the realization of an electrical contact of the conducting lines 20 to 24 to the contact areas 40 to 43.

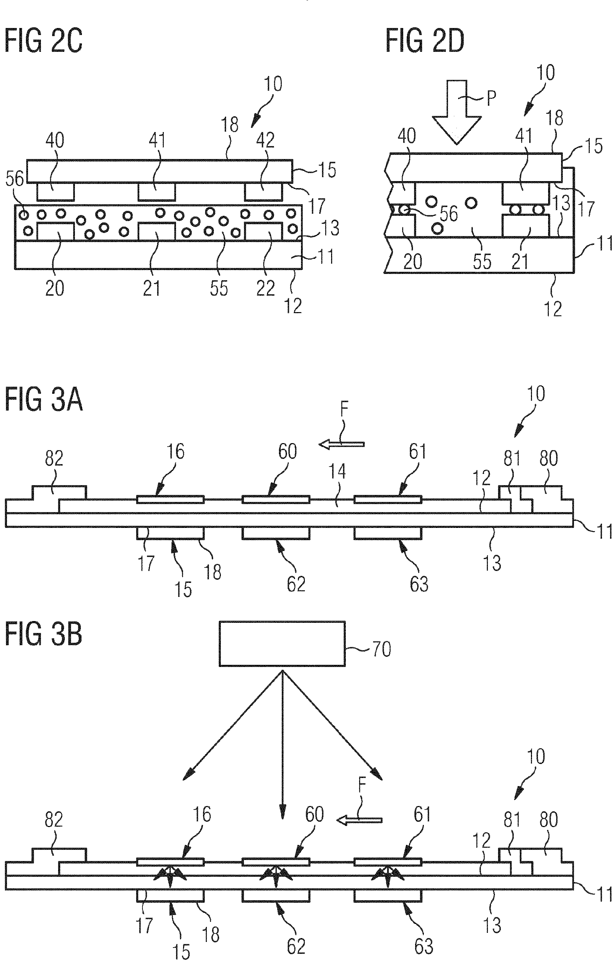

Figures 2C and 2D show details of a cross-section of the test strip 10 that is a further development of the test strip shown in the figures above. In the example shown in Figure 2C, the first to the third conducting lines 20 to 22 are fixed on the second side 13 of the substrate 11. The test strip 10 may comprise further conducting lines, not shown.

The surface of the conducting lines 20 to 22 rises above the surface of the second side 13 of the substrate 11.

The photodetector 15 is implemented as an integrated circuit, integrated circuit chip or integrated circuit die. In the example shown in Figure 2C, the photodetector 15 comprises the first to the third contact area 40 to 42. The photodetector 15 may comprise further contact areas. The contact areas 40 to 42 are realized as chip bumps. Thus, the surface of the contact areas 40 to 42 rises above the other parts of the surface of the photodetector 15.

Moreover, the test strip 10 comprises a film 55 that is arranged at the second side 13 of the substrate 11. The film 55 is located at the conducting lines 20 to 22. Thus, the film 55 is located at the surfaces of the conducting lines 20 to 22 and on those areas of the second side 13 of the substrate 11 which are not covered by the conducting lines 20 to 22. The film 55 may be an adhesive film. The film 55 is realized as an anisotropic conducting film. The film 55 comprises a non-conducting polymer and conducting particles 56. The particles may be realized as spheres.

In an alternative embodiment, not shown, the film 55 is arranged at the first side 17 of the photodetector 15. Thus, the film 55 is located at the surfaces of the contact areas 40 to 42 (that means at the surfaces of the chip bumps) and also on the areas on the first side 17 of the photodetector 15 which are not covered by the contact areas 40 to 42.

Figures 2C and 2D show the test strip 10 before and after the process step of fixating the photodetector 15 to the substrate 11. Whereas in Figure 2C the test strip 10 is shown before attachment of the photodetector 15 to the substrate 11, the test strip 10 is shown after the fixation of the photodetector 15 to the substrate 11 in Figure 2D.

As shown in Figure 2D, during the attachment process of the photodetector 15 to the substrate 11, pressure P or force is applied to a stack including the photodetector 15 and the substrate 11. Thus, the film 55 is squeezed. The stack may be heated during the attachment process. The conducting particles 56 may have a thin outer insulating layer which is broken by the pressure P. Thus, conducting particles 56, which are located between the contact areas 50 to 52 of the photodetector 15 and the conducting lines 20 to 22 on the substrate 11, form an electrical contact of the contact areas 50 to 52 to the conducting lines 20 to 22. There is at least one conducting particle 56 between a contact area 40 to 42 and the corresponding conducting line 20 to 22. The density of conducting particles 56 in the film 55 is so small that there is no lateral electrical connection of one contact area to an adjacent contact area via the conducting particles 56. Thus, the contact areas 40 to 42 are isolated from each other and, correspondingly, also the conducting lines 20 to 22 are electrically isolated from each other. The film 55 fills the gap between the photodetector 15 and the substrate 11.

The photodetector 15, also called spectral sensor, is directly integrated on the substrate 11. The substrate 11 is realized as plastic carrier. The photodetector 15 is attached with flip chip assembly technology or direct chip attach technology. The integrated circuit of the photodetector 15 is assembled in a way that the photodetector 15 is facing toward the nitrocellulose membrane through the carrier material (PET) 14 or any active area that need to be analyzed. By doing this it will be possible to control with high accuracy the alignment and optical between area that need to be analyzed and spectral sensor (basically they are all on the test strip 10). This will allow to increase sensitivity and signal-to-noise ratio during optical measurement, at the same time it will also simplify the hardware implementation of the PoC reader device. By having the photodetector 15 directly aligned with the active area 16 on the test strip 10, most of the photons/light going through the active area 16 of nitrocellulose will be focused or caught by the photodetector 15. Contrary to that, in a traditional system based on reflection measurements, the light/photons are scattered by the active areas and captured by the photodetector located in the same plane of the light source (in this case, if the test stripe is not perfectly aligned with the light source and the photodetector, then the photodetector may miss scattered energy and loses sensitivity).

The photodetector 15 is attached to the substrate 11 by using the anisotropic conducting film / glue (ACF) by applying small heat (depending by machine process and used ACF) and pressure to it. The photodetector 15 does not require any packaging in order to be directly attached on the test strip 10.

Figure 3A shows a further example of the test strip 10 that is a further development of the above-shown examples. In Figure 3A, a cross-section of the test strip 10 is shown. The test strip 10 is configured with multiple spectral sensors and multiple active areas. The porous material 14 comprises the active area 16 and an additional active area 60. Additionally, the porous material 14 may comprise a further active area 61. Thus, the porous material 14 may comprise a first number L of active areas. In the example shown in Figure 3A, the first number L is 3. The first number L may be larger than 1, larger than 2 or larger than 3. The first number L of active areas 16, 60, 61 may be rectangular, quadratic, circular or elliptical. The first number L of active areas 16, 60, 61 may reach e.g. from one border of the porous material 14 to the other border of the porous material 14.

Correspondingly, the test strip 10 comprises the first number L of photodetectors 15, 61, 62. The test strip 10 comprises the photodetector 15 and an additional photodetector 62 which is attached to the second side 13 of the substrate 11. Moreover, the test strip 10 may comprise a further photodetector 63 fixed to the second side 12 of the substrate 11.

The photodetector 15 is located adjacent to the active area 16 such that the photodetector 15 receives light from the active area 16. Correspondingly, the additional photodetector 62 is located adjacent to the additional active area 60. The further photodetector 63 is positioned adjacent to the further active area 61.

Thus, a separate photodetector 15, 62, 63 is located adjacent to each of the first number L of active areas 16, 60, 61. The additional and the further photodetector 62, 63 are implemented as spectral sensors. In the cross-section shown in Figure 3A, the flow F of the liquid in the porous material 14 is from the right side to the left side.

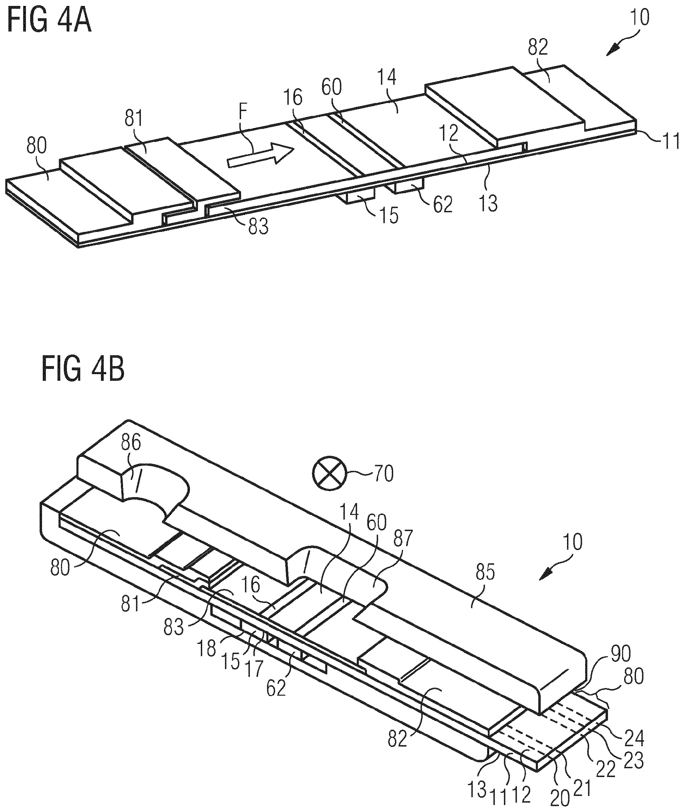

The test strip 10 comprises a sample pad 80, a conjugate pad 81 and an absorbent pad 82. The sample pad 80, the conjugate pad 81 and the absorbent pad 82 are arranged at the first side 12 of the substrate 11 and enclose the side surfaces of the porous material 14. The pads 80 to 82 will be further explained with respect to Figure 4A.

Figure 3B shows a further example of the test strip 10 that is based on the examples of the test strip shown in Figures 1, 2C, 2D and 3A and a light source 70. The light source 70 emits light. The light source 70 may be implemented as a spectral source. The light is emitted in a broad angle by the light source 70 and reaches the active area 16. Light of the light source 70 also reaches the additional and the further active area 60, 61. Thus, light emitted by the light source 70 reaches the first number L of active areas 16, 60, 61. The light source 70 may be fabricated as a broad band spectral source or black body spectral source, abbreviated BB spectral source. The light source 70 may emit light e.g. in the range from 390 nm to 1050 nm or from 390 nm to 750 nm. Alternatively, the light source 70 may be realized as a narrow band source (having e.g. a very limited bandwidth, such as for example a specific light color source or a pure near infrared source).

The substrate 11 is translucent or transparent. Additionally, the porous material 14 is translucent or transparent. Light from the active area 16 reaches the photodetector 15. The optical characteristic of the active area 16 depends on the optical characteristics of the porous material 14, of the chemical substances fixed at the porous material 14 in the active area 16 and of the concentration of an analyte in the liquid. For example, the light source 70 emits light in a broad spectrum. The active area 16 transmits light only in a small spectrum such as, for example, red light. The amount of light emitted or transmitted by the active area 16 depends on the concentration of the analyte in the liquid. The value of the light may rise with rising concentration of the analyte. The light transmitted by the active area 16 is detected by the photodetector 15.

The additional photodetector 62 and the further photodetector 63 can detect light originating from the additional and the further active area 60, 61. The first number L of active areas 16, 60, 61 may have different substances for reaction with the analytes of the liquid. Thus, the optical characteristics of the first number L of active areas 16, 60, 61 are different and are detected by the first number L of photodetectors 15, 62, 63.

Each of the first number L of photodetectors 15, 62, 63 may comprise a photodetector array 31, similar to Figures 2A and 2B. Thus, each of the first number L of photodetectors 15, 62, 63 is configured to detect light emitted by the first number L of active areas 16, 60, 61 at different ranges of light spectrum. The first number L of photodetectors 15, 62, 63 is realized using separate integrated circuits or chips or dies. The test strip 10 is fabricated with multiple spectral sensors and multiple active areas.

The detection/measurement is not realized as reflected ray measurements, but will be a refracted/transmitted/absorbed measurement. The light source 70 will be placed on one side of the test strip 10 and the detection is done on the other side of the substrate 11 of the test strip 10 by the photodetectors 15, 62, 63.

In an alternative embodiment, not shown, light is emitted by the light source 70 at one wavelength and is absorbed by a substance in the active area 16, wherein the substance in the active area 16 emit light at another wavelength. Thus, the light is emitted by the active area 16 using fluorescence or phosphorescence. In this case, the light source 70 emits light in a small band. The amount of light emitted by the active area 16 depends on the amount of analyte that reacts with the substance fixed at the active area 16. For example, the light source 70 is realized as a laser, a light-emitting diode or a vertical-cavity surface-emitting laser, abbreviated as VCSEL.

In an alternative embodiment, not shown, the light source 70 is omitted. Thus, a monitoring device is free from a light source. A reaction of a chemical substance of the active area 16 with an analyte in the liquid results in an emission of light. Thus, light is emitted by the active area 16 using chemo-luminescence.

In an alternative embodiment, not shown, the first number L of photodetectors 15, 62, 63 are realized on one die or chip. The first number L of photodetectors 15, 62, 63 may be implemented as pixels 30 or as photodetector arrays 31 on one die or chip.

Figure 4A shows an additional example of the test strip 10 which is a further development of the above-shown examples. The porous material 14 comprises the active area 16 and the additional active area 60. The active area 16 may be realized as a test active area, e.g. as a test line. The additional active area 60 may be implemented as a control active area, e.g. as a control line. Thus, a change of the optical characteristics at the additional active area 60 indicates that the test is performed correctly, for example that a sufficient amount of liquid has been provided to the test strip 10. A result of the test is detected by measurement of the optical characteristics at the active area 16. The substrate 11 may be fabricated as a backing card, for example as a plastic adhesive backing card.

Additionally, the test strip 10 comprises the sample pad 80. The sample pad 80 is configured to receive the liquid e.g. from a user or a liquid dispenser. The sample pad 80 is located on the first side 12 of the substrate 11. Moreover, the test strip 10 comprises the conjugate pad 81. The conjugate pad 81 is realized for providing a substance to the liquid. The conjugate pad 81 is located on the first side 12 of the substrate 11. The conjugate pad 81 is arranged between the sample pad 80 and the porous material 14. An overlap of the sample pad 80 is on the conjugate pad 81. Thus, an effective transfer of liquid from the sample pad 80 to the conjugate pad 81 is achieved using the overlap. An overlap of the conjugate pad 81 is on the porous material 14. An efficient transfer of liquid from the conjugate pad 81 to the porous layer 14 is achieved by the area of the overlap. The sample pad 80 and the conjugate pad 81 are located at a first end of the test strip 10.

The test strip 10 comprises the absorbent pad 82 being located on the first side 12 of the substrate 11. The absorbent pad 82 is arranged at a second end of the test strip 10. The absorbent pad 82 is in contact with the porous material 14. The absorbent pad 82 has an overlap with the porous material 14. Thus, a transfer of liquid is achieved from the porous material 14 to the absorbent pad 82 by the overlap. As indicated by the arrow F, the liquid flows from the sample pad 80 via the conjugate pad 81 and the porous material 14 to the absorbent pad 82. The liquid inserted on the sample pad 80 only partially reaches the absorbent pad 82. The photodetectors 15, 62 detect the change of the optical characteristics at the active areas 16, 60.

In general, the test strip 10 is built in a staked structure as following: The test strip 10 includes the substrate 11 which may be called carrier. The material of the substrate 11 is made e.g. of polystyrene, vinyl or polyester. In general, the substrate 11 is clear (that means transparent) or can be opaque too. An opaque substrate 11 may comprise a transparent or translucent window at the active area 16. The window may be realized by inserting a transparent or translucent material or by reducing the thickness of the substrate 11. The substrate 11 is used to hold the nitrocellulose membrane 14. Then on one side or end of the membrane 14 a detection conjugate is placed on the conjugate pad 81 followed by the sample pad 80. On the other side or end of the membrane 14, the absorbent pad 82 is placed.

The two lines, control line and test line 16, 60, show respectively the validity of the test and the test result. The disclosure refers how the carrier material is prepared prior nitrocellulose integration.

Figure 4B shows an example of the test strip 10 which is a further development of the above-shown examples. The test strip 10 comprises a housing 85. The housing 85 may be fabricated as plastic holder. The liquid can be inserted via a sample port 86 of the housing 85 to the sample pad 80. The housing 85 has at least one test opening 87. Thus, light emitted by the light source 70 can be transmitted via the test opening 87 to the active area 16 and to the additional active area 60. The test opening 87 has a form such that light emitted by the light source 70 reaches the number L of test areas 16, 60.

The test strip 10 comprises the conducting lines 20 to 24 which can be contacted at a connecting area 89. For example, the conducting lines 20 to 24 can be contacted at an end of the substrate 11. Thus, the housing 85 comprises an opening 90 such that a part of the substrate 11 is located outside of the housing 85.

In an alternative embodiment, not shown, the opening 90 of the housing 85 is on a bottom side of the housing 85. Thus, contacts to the conducting lines 20 to 24 are realized from below. Thus, the sample port 86 and the test opening 87 are located on an upper side of the housing 85 and the opening 90 is realized on a lower side of the housing 85.

In an alternative embodiment, not shown, the housing 85 comprises an additional opening. Light emitted by the light source 70 can be transmitted through the test opening 87 to the active area 16 and through the additional opening to the additional active area 60.

Figure 4C shows an example of a monitoring device 100 that comprises the light source 70. The test strip 10 as elucidated above can be inserted into the monitoring device 100 and can be removed again. The monitoring device 100 may be named reader or PoC reader. The monitoring device 100 includes a device housing 101. The light source 70 and part of the test strip 10 are located in the device housing 101.

In Figure 4C, the monitoring device 100 is only drawn schematically and as an example.

The monitoring device 100 may comprise a socket 103 having pins or spring contacts. The pins or spring contacts of the socket 103 contact the conducting lines 20 to 24. The monitoring device 100 may comprise guiding parts (not shown) to guide the test strip 10 e.g. into the socket 103. The device housing 101 may include parts to provide a light shield which shields light from the external of the monitoring device 100 from penetrating into the interior of the device housing 101.

Moreover, the monitoring device 100 comprises a control circuit 102 that is connected to the light source 70 and via the socket 102 and the conducting lines 20 to 24 to the at least one photodetector 15, 62, 63. The control circuit 102 is configured to detect whether the test strip 10 is inserted or not and to provide an enable signal, when the test strip 10 is inserted. Additionally, the monitoring device 100 may comprise an interface 104 connected to the control circuit 102 for providing information gained by the monitoring device 100 to an external device. The monitoring device 100 may also comprise a display 105 for displaying information gained by the control circuit 102. For example, the display 105 may display the enable signal to indicate to a user that the sample liquid can be applied to the test strip 10. Moreover, the display 105 displays the result of the test. The monitoring device 100 may comprise a power supply 106 such as a battery. Additionally, the monitoring device 100 may comprise a user interface 107 such as a button to start the measuring process.

The monitoring device 100 is free of a complicate mechanical switch for detecting the test strip insertion into the monitoring device 100. The test strip 10 will have electrical connection (termination) corresponding to the various signal and power lines on the substrate 11. The electronics on the monitoring device 100 will be able to detect the insertion of the test strip 10 by detecting the spectral sensor 15 on the I²C lines and consequently enable the monitoring device 100 in a way that is ready to run measurements.

Optionally, a protection system can be implemented on the I²C line to avoid that counterfeiting strips are inserted. The test strip 10 may be masked in a way that only electronic reading is allowed. This can be done by completely covering the area where the photodetectors 15, 62, 63 are located or both side leaving only a small slit to allow the impinging light to reach the membrane 14.

The test strip 10 may be configured to perform reactance measurements. To achieve proper measurements the test strip 10 may be equipped with a qualified spectrometer photodetector integrated circuit. The test strip 10 may be named test stripe.

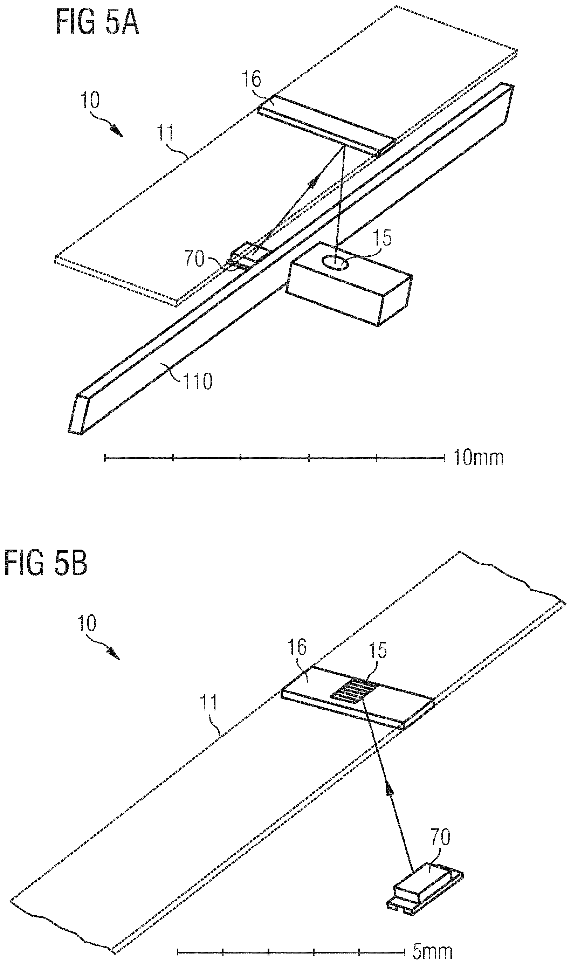

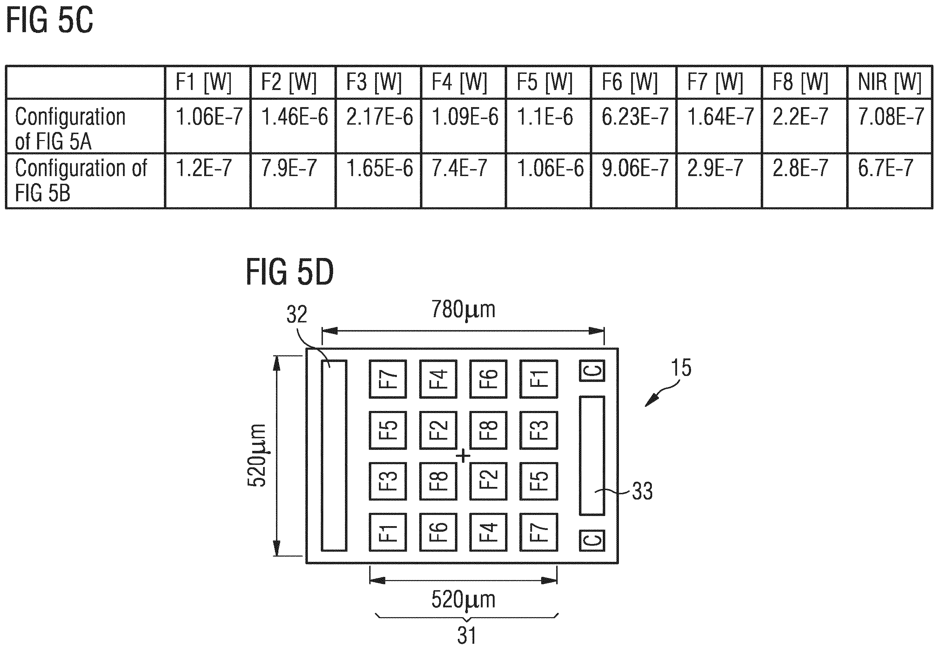

Figures 5A and 5B show two different schematic configurations of a monitoring device 100, Figure 5C shows results of the simulation and Figure 5D shows an example of the photodetector 15 that has been used for simulation.

In Figure 5A, a reflective configuration is illustrated that does not form part of the present invention. Light emitted by the light source 70 hits the active area 16 on the test strip 10. Light reflected or emitted by the active area 16 is detected by the photodetector 15. The light source 70 and the photodetector 15 are approximately in the same plane; the active area 16 is above the plane. A light barrier 110 protects the photodetector 15 from light generated by the light source 70. The light source 70 may be a light-emitting diode, e.g. emitting white light or broad band white light. An example of a light path is shown in Figure 5A.

In Figure 5B, a configuration as shown in Figures 3B, 4B or 4C is illustrated. Light emitted by the light source 70 reaches the photodetector 15 via the active area 16 on the test strip 10. The light source 70 may be a light-emitting diode, e.g. emitting white light or broad band white light. An example of a light path is shown in Figure 5B.

In Figure 5C, simulation results of the configurations shown in Figures 5A and 5B are listed. As shown in Figure 5D, the photodetector 15 has the photodetector array 31 with sixteen pixels 30. The photodetector array 31 includes eight pairs of pixels 30, indicated by F1 to F8. The pixels 30 of a pair are sensitive for the same wavelength region in visible light. The additional pixel 32 detects a flicker of ambient light. The further pixel 33 is sensitive in the near-infrared range (abbreviated NIR range). The table in Figure 5C shows the simulation results as total power in Watt for the eight pairs F1 to F8 and the NIR sensitive further pixel 33. As shown in the table, four pairs of pixels F2 to F5 and the NIR sensitive further pixel 33 achieve better results in the configuration of Figure 5A and also four pairs of pixels F1, F6 to F8 achieve better results in the configuration of Figure 5B. The differences are small. Thus, both configurations achieve comparable simulation results. For both configurations, a wort case distance between the light source 70 and the photodetector 15 on one side and the active area 16 on the other side is approximately 5 mm.

The embodiments shown in Figures 1 to 5D as stated represent example embodiments of the improved test strip, the monitoring device and method for fabricating a test strip, therefore they do not constitute a complete list of all embodiments according to the improved test strip, the monitoring device and method. Actual test strips and methods for fabricating may vary from the embodiments shown in terms of devices, layers, shape, size and materials, for example.

### List of Reference Signs

- 10: test strip
- 11: substrate
- 12: first side
- 13: second side
- 14: porous material
- 15: photodetector
- 16: active area
- 17: first side
- 18: second side
- 20 to 24: conducting line
- 30: pixel
- 31: photodetector array
- 32, 33: further pixel
- 40 to 46: contact area
- 51 to 54: border
- 55: anisotropic conducting film
- 56: conducting particle
- 60, 61: active area
- 62, 63: photodetector
- 70: light source
- 80: sample pad
- 81: conjugate pad
- 82: absorbent pad
- 83: input point
- 85: housing
- 86: sample port
- 87: test opening
- 89: connecting area
- 90: opening
- 100: monitoring device
- 101: device housing
- 102: control circuit
- 103: socket
- 104: interface
- 105: display
- 106: power supply
- 107: user interface
- 110: light barrier
- D, D1, D2: distance
- F: flow
- GND: reference potential
- INT: signal
- P: pressure
- SCL: clock signal
- SDA: data signal
- VCC: supply voltage

## Claims

1. Test strip, comprising
- a porous material (14), wherein the porous material (14) is configured to transfer a liquid from an input point of the porous material (14) to an active area (16) of the porous material (14), and wherein the active area (16) is provided with a chemical substance which reacts with a component of the liquid,
- a photodetector (15), and
- a substrate (11) with a first and a second side (12, 13), wherein the porous material (14) is attached to the first side (12) of the substrate (11) and the photodetector (15) is attached to the second side (13) of the substrate (11) adjacent to the active area (16),
**characterized in that**
- the substrate (11) is made of plastic or a polymer or glass,
- the photodetector (15) is implemented as a spectral sensor that is configured to separately detect light in at least two different wavelength regions, wherein two respective colors are generated at the active area (16) by respective reactions with two components of the liquid, each of the colors being measured separately by the spectral sensor, and
- the test strip is free from a light source (70).

2. Test strip of claim 1,
wherein the test strip (10) comprises conducting lines (20 to 24) arranged at the second side (13) of the substrate (11), and
wherein the photodetector (15) comprises contact areas (40 to 44) which are electrically connected to the conducting lines (20 to 24) at the second side (13) of the substrate (11) .

3. Test strip of claim 2,
wherein the test strip (10) comprises an anisotropic conducting film (55) that mechanically connects the photodetector (15) to the second side (13) of the substrate (11) and electrically connects the contact areas (40 to 44) of the photodetector (15) to the conducting lines (20 to 24) at the second side (13) of the substrate (11) .

4. Test strip of claim 2 or 3,
wherein the conducting lines (20 to 24) comprise a power supply line (20), a reference potential line (21) and at least one bus line (22, 23).

5. Test strip of one of claims 1 to 4,
wherein the substrate (11) is transparent or translucent, or
the substrate (11) is opaque and comprises a transparent or a translucent area.

6. Test strip of one of claims 1 to 5,
wherein the porous material (14) comprises nitrocellulose.

7. Test strip of claim 1,
wherein the porous material (14) comprises at least two active areas (16, 60) and
wherein the photodetector (15) comprises at least two pixels which are arranged adjacent to the at least two active areas (16, 60) or
the photodetector (15) is arranged adjacent to one of the at least two active areas (16, 60) and an additional photodetector (62) of the test strip (10) is arranged adjacent to another one of the at least two active areas (16, 60) .

8. Monitoring device, comprising
- a test strip (10) comprising
- a porous material (14), wherein the porous material (14) is configured to transfer a liquid from an input point of the porous material (14) to an active area (16) of the porous material (14), and wherein the active area (16) is provided with a chemical substance which reacts with a component of the liquid,
- a photodetector (15), wherein the photodetector (15) is implemented as a spectral sensor that is configured to separately detect light in at least two different wavelength regions, wherein two respective colors are generated at the active area (16) by respective reactions with two components of the liquid, each of the colors being measured separately by the spectral sensor, and
- a substrate (11) with a first and a second side (12, 13), wherein the porous material (14) is attached to the first side (12) of the substrate (11) and the photodetector (15) is attached to the second side (13) of the substrate (11) adjacent to the active area (16), wherein the substrate (11) is made of plastic or a polymer or glass,
- a light source (70), and
- a control circuit (102) connected to the photodetector (15) and to the light source (70),
wherein the porous material (14) and the substrate (11) are located between the light source (70) and the photodetector (15).

9. Monitoring device of claim 8,
wherein the monitoring device (100) is operable such that the test strip (10) is inserted into the monitoring device (100) and removed again.

10. Monitoring device of claim 8 or 9,
wherein the control circuit (102) is operable to detect whether the test strip (10) is inserted or not and to provide an enable signal, when the test strip (10) is inserted.

11. Monitoring device of one of claims 8 to 10,
wherein the monitoring device (100) is operable to perform a lateral flow test or a lateral flow immunochromatographic assay.

12. Method for fabricating a test strip, comprising
- providing a substrate (11) with a first and a second side (12, 13),
- attaching a porous material (14) to the first side (12) of the substrate (11), wherein the porous material (14) is configured to transfer a liquid from an input point of the porous material (14) to an active area (16) of the porous material (14), and wherein the active area (16) is provided with a chemical substance which reacts with a component of the liquid, and
- attaching a photodetector (15) to the second side (13) of the substrate (11) adjacent to the active area (16),
**characterized in that**
- the substrate (11) is made of plastic or a polymer or glass,
- the photodetector (15) is implemented as a spectral sensor that is configured to separately detect light in at least two different wavelength regions, wherein two respective colors are generated at the active area (16) by respective reactions with two components of the liquid, each of the colors being measured separately by the spectral sensor, and
- the test strip is free from a light source (70).

13. Method of claim 12,
wherein the photodetector (15) is attached to the second side (13) of the substrate (11) by flip-chip assembly.

14. Method of claim 12 or 13,
wherein an anisotropic conducting film (55) is provided between the photodetector (15) and the second side (13) of the substrate (11).

## Patentansprüche

1. Teststreifen, umfassend
- ein poröses Material (14), wobei das poröse Material (14) so ausgebildet ist, dass es eine Flüssigkeit von einem Eingangspunkt des porösen Materials (14) zu einem aktiven Bereich (16) des porösen Materials (14) überträgt, und wobei der aktive Bereich (16) mit einer chemischen Substanz versehen ist, die mit einer Komponente der Flüssigkeit reagiert,
- einen Fotodetektor (15), und
- ein Substrat (11) mit einer ersten und einer zweiten Seite (12, 13), wobei das poröse Material (14) an der ersten Seite (12) des Substrats (11) angebracht ist und der Fotodetektor (15) an der zweiten Seite (13) des Substrats (11) angrenzend an den aktiven Bereich (16) angebracht ist,
**dadurch gekennzeichnet, dass**
- das Substrat (11) aus Kunststoff oder einem Polymer oder Glas hergestellt ist,
- der Fotodetektor (15) als Spektralsensor ausgeführt ist, der so ausgebildet ist, dass er Licht in mindestens zwei unterschiedlichen Wellenlängenbereichen getrennt detektiert, wobei an dem aktiven Bereich (16) durch jeweilige Reaktionen mit zwei Komponenten der Flüssigkeit jeweils zwei Farben erzeugt werden, die vom Spektralsensor jeweils getrennt gemessen werden, und
- der Teststreifen frei von einer Lichtquelle (70) ist.

2. Teststreifen nach Anspruch 1,
wobei der Teststreifen (10) an der zweiten Seite (13) des Substrats (11) angeordnete Leiterbahnen (20 bis 24) aufweist, und
wobei der Fotodetektor (15) Kontaktflächen (40 bis 44) aufweist, die mit den Leiterbahnen (20 bis 24) an der zweiten Seite (13) des Substrats (11) elektrisch verbunden sind.

3. Teststreifen nach Anspruch 2,
wobei der Teststreifen (10) einen anisotropen leitenden Film (55) umfasst, der den Fotodetektor (15) mechanisch mit der zweiten Seite (13) des Substrats (11) verbindet und die Kontaktbereiche (40 bis 44) des Fotodetektors (15) elektrisch mit den Leiterbahnen (20 bis 24) an der zweiten Seite (13) des Substrats (11) verbindet.

4. Teststreifen nach Anspruch 2 oder 3,
wobei die Leiterbahnen (20 bis 24) eine Stromversorgungsleitung (20), eine Bezugspotentialleitung (21) und mindestens eine Busleitung (22, 23) umfassen.

5. Teststreifen nach einem der Ansprüche 1 bis 4,
wobei das Substrat (11) transparent oder durchscheinend ist, oder
das Substrat (11) lichtundurchlässig ist und einen transparenten oder durchscheinenden Bereich aufweist.

6. Teststreifen nach einem der Ansprüche 1 bis 5,
wobei das poröse Material (14) Nitrocellulose umfasst.

7. Teststreifen nach Anspruch 1,
wobei das poröse Material (14) mindestens zwei aktive Bereiche (16, 60) aufweist und
wobei der Fotodetektor (15) mindestens zwei Pixel aufweist, die benachbart zu den mindestens zwei aktiven Bereichen (16, 60) angeordnet sind oder
der Fotodetektor (15) benachbart zu einem der mindestens zwei aktiven Bereiche (16, 60) angeordnet ist und ein weiterer Fotodetektor (62) des Teststreifens (10) benachbart zu einem anderen der mindestens zwei aktiven Bereiche (16, 60) angeordnet ist.

8. Messgerät, das Folgendes umfasst
- einen Teststreifen (10) umfassend
- ein poröses Material (14), wobei das poröse Material (14) so ausgebildet ist, dass es eine Flüssigkeit von einem Eingangspunkt des porösen Materials (14) zu einem aktiven Bereich (16) des porösen Materials (14) leitet, und wobei der aktive Bereich (16) mit einer chemischen Substanz versehen ist, die mit einer Komponente der Flüssigkeit reagiert,
- einen Fotodetektor (15), wobei der Fotodetektor (15) als Spektralsensor ausgebildet ist, der so ausgelegt ist, dass er Licht in mindestens zwei verschiedenen Wellenlängenbereichen separat detektiert, wobei an dem aktiven Bereich (16) durch entsprechende Reaktionen mit zwei Komponenten der Flüssigkeit jeweils zwei Farben erzeugt werden, die jeweils separat von dem Spektralsensor gemessen werden, und
- ein Substrat (11) mit einer ersten und einer zweiten Seite (12, 13), wobei das poröse Material (14) an der ersten Seite (12) des Substrats (11) angebracht ist und der Fotodetektor (15) an der zweiten Seite (13) des Substrats (11) angrenzend an den aktiven Bereich (16) angebracht ist, wobei das Substrat (11) aus Kunststoff oder einem Polymer oder Glas hergestellt ist,
- eine Lichtquelle (70), und
eine Steuerschaltung (102), die mit dem Fotodetektor (15) und der Lichtquelle (70) verbunden ist,
wobei das poröse Material (14) und das Substrat (11) zwischen der Lichtquelle (70) und dem Fotodetektor (15) angeordnet sind.

9. Messgerät nach Anspruch 8,
wobei das Messgerät (100) derart betreibbar ist, dass der Teststreifen (10) in das Messgerät (100) eingeführt und wieder entnommen werden kann.

10. Messgerät nach Anspruch 8 oder 9,
wobei die Steuerschaltung (102) betreibbar ist, um zu erkennen, ob der Teststreifen (10) eingeführt ist oder nicht, und um ein Freigabesignal bereitzustellen, wenn der Teststreifen (10) eingeführt ist.

11. Messgerät nach einem der Ansprüche 8 bis 10,
wobei das Messgerät (100) zur Durchführung eines Lateralfluss-Tests oder eines immunochromatographischen Lateralfluss-Assays betreibbar ist.

12. Verfahren zum Herstellen eines Teststreifens, umfassend
- Bereitstellen eines Substrats (11) mit einer ersten und einer zweiten Seite (12, 13),
- Anbringen eines porösen Materials (14) an der ersten Seite (12) des Substrats (11), wobei das poröse Material (14) so ausgebildet ist, dass es eine Flüssigkeit von einem Eingangspunkt des porösen Materials (14) zu einem aktiven Bereich (16) des porösen Materials (14) überträgt, und wobei der aktive Bereich (16) mit einer chemischen Substanz versehen ist, die mit einer Komponente der Flüssigkeit reagiert, und
- Anbringen eines Fotodetektors (15) an der zweiten Seite (13) des Substrats (11) neben dem aktiven Bereich (16), **dadurch gekennzeichnet, dass**
- das Substrat (11) aus Kunststoff, einem Polymer oder Glas hergestellt ist,
- der Fotodetektor (15) als Spektralsensor ausgeführt ist, der so ausgebildet ist, dass er Licht in mindestens zwei verschiedenen Wellenlängenbereichen separat detektiert, wobei an dem aktiven Bereich (16) durch entsprechende Reaktionen mit zwei Komponenten der Flüssigkeit jeweils zwei Farben erzeugt werden, die jeweils separat von dem Spektralsensor gemessen werden, und
- der Teststreifen frei von einer Lichtquelle (70) ist.

13. Verfahren nach Anspruch 12,
wobei der Fotodetektor (15) auf der zweiten Seite (13) des Substrats (11) durch Flip-Chip-Montage angebracht wird.

14. Verfahren nach Anspruch 12 oder 13,
wobei ein anisotroper leitender Film (55) zwischen dem Fotodetektor (15) und der zweiten Seite (13) des Substrats (11) bereitgestellt ist.

## Revendications

1. Bande de test, comprenant
- un matériau poreux (14), dans lequel le matériau poreux (14) est configuré pour transférer un liquide d'un point d'entrée du matériau poreux (14) à une zone active (16) du matériau poreux (14), et dans lequel la zone active (16) est pourvue d'une substance chimique qui réagit avec un composant du liquide,
- un photodétecteur (15), et
- un substrat (11) avec un premier et un second côté (12, 13), dans lequel le matériau poreux (14) est fixé au premier côté (12) du substrat (11) et le photodétecteur (15) est fixé au second côté (13) du substrat (11) adjacent à la zone active (16),
**caractérisé par le fait que**
- le substrat (11) est fabriqué en plastique ou en polymère ou en verre,
- le photodétecteur (15) est mis en oeuvre comme un capteur spectral configuré pour détecter séparément la lumière dans au moins deux régions de longueur d'onde différentes, dans lequel deux couleurs respectives sont générées dans la zone active (16) par des réactions respectives avec deux composants du liquide, chacune des couleurs étant mesurée séparément par le capteur spectral, et
- la bande de test est libre de toute source de lumière (70).

2. Bande de test de la revendication 1,
dans laquelle la bande de test (10) comprend des lignes conductrices (20 à 24) disposées sur la deuxième côté (13) du substrat (11), et
dans laquelle le photodétecteur (15) comprend des zones de contact (40 à 44) qui sont électriquement connectées aux lignes conductrices (20 à 24) au niveau du deuxième côté (13) du substrat (11).

3. Bande de test de la revendication 2,
dans laquelle la bande de test (10) comprend un film conducteur anisotrope (55) qui connecte mécaniquement le photodétecteur (15) au deuxième côté (13) du substrat (11) et connecte électriquement les zones de contact (40 à 44) du photodétecteur (15) aux lignes conductrices (20 à 24) au niveau du deuxième côté (13) du substrat (11).

4. Bande de test de la revendication 2 ou 3,
dans laquelle les lignes conductrices (20 à 24) comprennent une ligne d'alimentation (20), une ligne de potentiel de référence (21) et au moins une ligne de bus (22, 23).

5. Bande de test de l'une des revendications 1 à 4, dans laquelle le substrat (11) est
transparent ou translucide, ou
le substrat (11) est opaque et comprend une zone transparente ou translucide.

6. Bande de test de l'une des revendications 1 à 5,
dans laquelle le matériau poreux (14) comprend nitrocellulose.

7. Bande de test de la revendication 1,
dans laquelle le matériau poreux (14) comprend au moins deux zones actives (16, 60) et
dans laquelle le photodétecteur (15) comprend au moins deux pixels qui sont disposés à côté des au moins deux zones actives (16, 60) ou
le photodétecteur (15) est disposé à côté de l'une des au moins deux zones actives (16, 60) et un photodétecteur supplémentaire (62) de la bande de test (10) est disposé à côté d'une autre des au moins deux zones actives (16, 60).

8. Dispositif de contrôle comprenant
- une bande de test (10) comprenant
- un matériau poreux (14), dans lequel le matériau poreux (14) est configuré pour transférer un liquide d'un point d'entrée du matériau poreux (14) à une zone active (16) du matériau poreux (14), et dans lequel la zone active (16) est pourvue d'une substance chimique qui réagit avec un composant du liquide,
- un photodétecteur (15), dans lequel le photodétecteur (15) est mis en oeuvre comme un capteur spectral configuré pour détecter séparément la lumière dans au moins deux régions de longueur d'onde différentes, dans lequel deux couleurs respectives sont générées dans la zone active (16) par des réactions respectives avec deux composants du liquide, chacune des couleurs étant mesurée séparément par le capteur spectral, et
- un substrat (11) avec un premier et un second côté (12, 13), dans lequel le matériau poreux (14) est fixé au premier côté (12) du substrat (11) et le photodétecteur (15) est fixé au second côté (13) du substrat (11) adjacent à la zone active (16), dans lequel le substrat (11) est fabriqué en plastique ou en polymère ou en verre,
- une source lumineuse (70), et
- un circuit de commande (102) connecté au photodétecteur (15) et à la source lumineuse (70),
dans lequel le matériau poreux (14) et le substrat (11) sont disposés entre la source lumineuse (70) et le photodétecteur (15) .

9. Dispositif de contrôle de la revendication 8,
dans lequel le dispositif de contrôle (100) est utilisable de manière à ce que la bande de test (10) soit insérée dans le dispositif de contrôle (100) et en soit à nouveau retirée.

10. Dispositif de contrôle de la revendication 8 ou 9,
dans lequel le circuit de commande (102) est utilisable pour détecter si la bande de test (10) est insérée ou non et pour fournir un signal de validation lorsque la bande de test (10) est insérée.

11. Dispositif de contrôle de l'une des revendications 8 à 10,
dans lequel le dispositif de contrôle (100) est utilisable pour effectuer un test de flux latéral ou un test immunochromatographique de flux latéral.

12. Méthode de fabrication d'une bande de test, comprenant
- fournir un substrat (11) avec un premier et un second côté (12, 13),
- fixer un matériau poreux (14) au premier côté (12) du substrat (11), dans lequel le matériau poreux (14) est configuré pour transférer un liquide d'un point d'entrée du matériau poreux (14) à une zone active (16) du matériau poreux (14), et dans lequel la zone active (16) est pourvue d'une substance chimique qui réagit avec un composant du liquide, et
- fixer un photodétecteur (15) sur le deuxième côté (13) du substrat (11) adjacent à la zone active (16),
**caractérisé par le fait que**
- le substrat (11) est en plastique, en polymère ou en verre,
- le photodétecteur (15) est mis en œuvre comme un capteur spectral configuré pour détecter séparément la lumière dans au moins deux régions de longueur d'onde différentes, dans lequel deux couleurs respectives sont générées dans la zone active (16) par des réactions respectives avec deux composants du liquide, chacune des couleurs étant mesurée séparément par le capteur spectral, et
- la bande de test est libre de toute source de lumière (70).

13. Méthode de la revendication 12,
dans lequel le photodétecteur (15) est fixé sur le deuxième côté (13) du substrat (11) par assemblage flip-chip.

14. Méthode de la revendication 12 ou 13,
dans lequel un film conducteur anisotrope (55) est disposé entre le photodétecteur (15) et le second côté (13) du substrat (11).
